# EUROPEAN PATENT SPECIFICATION

(11) **EP 2 297 159 B1**
(45) Date of publication and mention of the grant of the patent: **15.08.2012**
(21) Application number: 09746113.1
(22) Date of filing: 13.05.2009
(51) Int. Cl.: C07D 487/04, A61K 31/5025, A61P 7/02

(54) **SUBSTITUTED QUINAZOLINES**
SUBSTITUIERTE CHINAZOLINE
QUINAZOLINES SUBSTITUÉS

(30) Priority: 16.05.2008 GB 0808947
(43) Date of publication of application: 23.03.2011
(73) Proprietor: Shire LLC, Florence, KY 41042 (US)
(72) Inventor: GOLDING, Bernard, T., Tyne and Wear NE27 0EZ (GB); FRANKLIN, Richard, Hampshire GU51 4NQ (GB); MACLEOD, Angus, Essex CB10 1XL (GB); CICALA, Peter, Wayne Pennsylvania 19087-8301 (US)
(74) Representative: Harrison Goddard Foote
(86) International application number: PCT/GB2009/050517
(87) International publication number: WO 2009/138796

(56) References cited:
- GB-A- 1 418 822
- JP-A- 2004 051 594
- US-A- 3 983 120
- US-A1- 2007 066 619
- VENUTI, MICHAEL C. ET AL: "Inhibitors of Cyclic AMP Phosphodiesterase. 3. Synthesis and Biological Evaluation of Pyrido and Imidazolyl Analogues of 1,2,3,5-Tetrahydro-2-oxoimidazo[2,1-b]quin azoline" JOURNAL OF MEDICINAL CHEMISTRY, vol. 31, no. 11, 1 November 1988 (1988-11-01), pages 2136-2145, XP002131132 ISSN: 0022-2623
- KIENZLE, FRANK ET AL: "1,5-Dihydroimidazoquinazolinones as blood platelet aggregation inhibitors" EUROPEAN JOURNAL OF MEDICINAL CHEMISTRY , 17(6), 547-56 CODEN: EJMCA5; ISSN: 0009-4374, 1982, XP002550118

## Description

### FIELD OF THE INVENTION

This invention relates to the discovery of substituted analogues of the selective platelet lowering agent anagrelide with reduced potential for cardiovascular side-effects which should lead to improved patient compliance and safety in the treatment of myeloproliferative diseases. More specifically, the present invention relates to certain imidazoquinazoline derivatives which have utility as platelet lowering agents in humans. The compounds of the present invention function by inhibiting megakaryocytopoeisis and hence the formation of blood platelets.

### BACKGROUND OF THE INVENTION.

Anagrelide hydrochloride (Agrylin®, Xagrid®) is a novel orally administered imidazoquinazoline which selectively reduces platelet count in humans and is used for such purposes in the treatment of myeloproliferative diseases (MPDs), such as essential thrombocythemia (ET), where an elevated platelet count may put the patient at increased thrombotic risk. The chemical structure of anagrelide, 6,7-dichloro-1,5-dihydroimidazo[2,1-*b*]-quinazolin-2(3*H*)-one is shown as the hydrochloride monohydrate in the following formula:

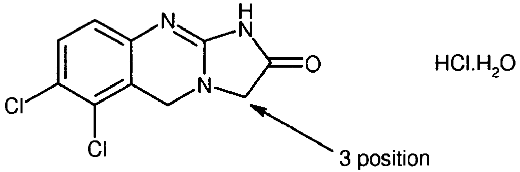

Preparation of anagrelide hydrochloride was referred to in U.S. Patent Nos. 3,932,407; RE31,617 and 4,146,718.

Anagrelide is a unique, highly selective platelet lowering agent. *In vitro* studies of human megakaryocytopoiesis suggested that, *in vivo,* its thrombocytopenic activity results primarily from an inhibitory effect on megakaryocyte maturation. Anagrelide inhibited TPO-induced megakaryocytopoiesis in a dose-dependent manner with an estimated IC₅₀ of ~26 nM, showing it to be a highly potent agent. Anagrelide does not affect erythroid or myelomonocytic differentiation stimulated by erythropoietin or granulocyte-macrophage colony-stimulating factor, demonstrating the selectivity of this compound against the megakaryocytic lineage.

The drug, which is available in both the U.S. and Europe, has proven to be of considerable clinical value in the treatment of myeloproliferative diseases, such as essential thrombocythemia. Anagrelide was shown to be effective and selective in reducing and maintaining platelet count close to or within the physiological range in patients with thrombocythemia secondary to a myeloproliferative disorder. The time to complete response, defined as a platelet count ≤ 600x10⁹/L, ranged from 4 to 12 weeks. In the majority of patients, the platelet count can be reduced and maintained at a dose of 1 to 3mg/day.

In early volunteer trials, the most frequently reported adverse effects AEs other than headache were palpitations, postural dizziness and nausea. During patient studies, the most frequently reported drug-related AEs were headache, palpitations, oedema/fluid retention, nausea/vomiting, diarrhea, dizziness and abdominal pain. These effects are all likely to arise from the secondary, cardiovascular pharmacology associated with anagrelide resulting from its inhibitory effects on human phosphodiesterase III (PDE III). Anagrelide is a potent PDE III inhibitor with an IC₅₀ value of ~29 nM (cf. milrinone, a classical PDE III inhibitor, IC₅₀ = 170-350 nM). Inhibition of myocardial PDE III leads to positive inotropy (increasing of the force of contractions of the heart), increased chronotropy (increase in heart rate), and peripheral vasodilatation. Such cardiovascular manifestations of this inhibition are typically seen with the classical positive inotropes, milrinone and enoximone, and exploited in the short-term acute treatment of congestive heart failure. However, in the treatment of a so-called silent disease (*i.e*., asymptomatic) such as ET, the cardiovascular side-effects of palpitations and tachycardia associated with anagrelide limit its utility and a significant proportion of patients - reportedly between 25 and 50% - fail to tolerate the drug during long term treatment.

The PDE III inhibitory properties of anagrelide are quite distinct from its platelet lowering antimegakaryocytic effects. Indeed studies have shown no correlation between potency as a PDE III inhibitor and anti-megakaryocytic effects for anagrelide and its principal pharmacologically active metabolite, 3-hydroxyanagrelide (3-OH anagrelide or 3-HA, formerly known as SPD604 or BCH24426). Surprisingly the latter was found to be over 40-fold more potent than anagrelide as a PDE III inhibitor. With respect to inhibition of megakaryocytopoiesis (and therefore platelet lowering potential) it was however no more potent than the parent drug. Anagrelide's active metabolite, 3-HA, is present *in vivo* in amounts greatly exceeding those of the parent drug with typical exposures being 2-3 fold greater. Thus by implication 3-OH anagrelide is likely to be a major contributor to the pharmacological actions of the drug.

In addition to the unwanted cardiovascular effects associated with PDE III inhibition, the consequent elevation of cAMP can result in an anti-aggregatory effect. While initially this property may appear to be beneficial in essential thrombocythemia patients predisposed to greater thrombotic risk, such anti-platelet effects, in excess, could have haemorrhagic consequences and on balance may not be desirable. Indeed the haemorrhagic events occasionally seen in ET patients treated with anagrelide might be due to a combination of the anti-aggregatory effects contributed largely by 3-OH anagrelide and an overshooting of platelet reduction, compounded by a synergistic interaction with aspirin that is frequently concomitantly administered. (In some ET patients, plasma concentrations of 3-OH anagrelide have been shown likely to exceed the *in vitro* IC₅₀ values for inhibition of platelet aggregation by a factor of 3).

The PDE III mediated cardiovascular side-effects associated with anagrelide treatment mean that many patients have to be switched to the only significant alternative therapy, namely that with hydroxyurea. However, this drug is a simple chemical anti-metabolite which inhibits ribonucleoside diphosphate reductase (RNR) with resultant profound effects on DNA synthesis. Ribonucleoside diphosphate reductase catalyzes the conversion of ribonucleosides into deoxyribonucleosides, which are the building blocks of DNA synthesis and repair. Inhibition of ribonucleoside diphosphate reductase explains the cytoreductive and - most importantly - the mutagenic effects of this compound as well as its platelet lowering action. Hydroxyurea is thus officially classified as a "presumed human carcinogen." As well as possessing the potential to induce leukemic transformation, hydroxyurea is associated with the induction of difficult-to-treat leg ulcers.

Faced with this dilemma in treatment options, there is a clear need for a new agent in the treatment of thrombocythemia which is selective in its effects on megakaryocytopoiesis but with reduced or minimal side effects. While anagrelide offers some selectivity in its mechanism of action, the limitations to its use are those associated with cardiovascular effects resulting from its secondary pharmacology and contributed largely by the active metabolite of anagrelide, 3-hydroxyanagrel ide.

The metabolism of anagrelide generally proceeds extremely rapidly, resulting in a less than ideal pharmacokinetic profile of the drug.. The typical half-life of anagrelide is just 1.5 hr (2.5 hr for the metabolite) necessitating frequent drug administration (up to 4 times per day). This, combined with the side-effects profile, can lead to poor patient compliance. Furthermore, anagrelide undergoes a large first pass effect (>50%) leading to considerable intersubject variation in achieved exposures and, therefore, potentially variable drug response. Also, exposure to the pharmacologically active metabolite varies dramatically between patients since its formation is dependent on CYP1A, an enzyme whose expression is highly dependent on exposure to inducing agents such as cigarette smoke. Overall, this may result in the need for careful dose titration in patients being treated with anagrelide.

US4256748 discloses a number of imidazo[2,1-b]quinazolin-2(3H)-ones which have an analogous structure to anagrelide and which are said to be effective in the treatment of thromboses resulting from their anti-aggregatory effects on blood platelets mediated by PDE III inhibition. However, this disclosure does not appreciate the entirely separate antimegakaryocytic potential (reducing platelet numbers) which could be associated with some analogues.

Similarly, Kienzle et al (Eur. J. Med. Chem., 17, 1982, 547-556) describe the use of 1,5-dihydroimidazoquinazolinones as blood platelet aggregation inhibitors.

Ideally there is a need for compounds which possess anti-megakaryocytic activity whilst at the same time having a reduced level of PDE III inhibitory activity and therefore unwanted cardiovascular effects.

It is an aim of the present invention to overcome various disadvantages of or to improve on the properties of prior art compounds. Thus it is an aim of the invention to provide an anagrelide derivative which has improved activity and / or reduced cardiovascular toxicity relative to prior art compounds in the treatment of diseases for which modulation of megakaryocytopoeisis provides an efficacious treatment. The compounds of the present invention are especially beneficial because they display less inhibitory activity towards phosphodiesterase III (PDE III) and yet surprisingly still retain their anti-megakarycocytic and hence platelet lowering properties.

It is also desirable that the compounds of the present invention should have an improved pharmacokinetic profile to aid patient compliance and ensure consistency of therapeutic response. It is thus a further aim to provide compounds with a good duration of action i.e. long half-life in vivo. Additionally it is a further aim to provide compounds that are available via relatively convenient synthetic processes.

The compounds described in relation to the present invention satisfy some or all of the above aims.

### SUMMARY OF THE INVENTION

We have found that analogues of anagrelide in which the principal site of metabolism is blocked by an appropriate group are likely not only to have improved pharmacokinetics but also a better side effect profile. This would be expected to lead to better tolerability and improved patient compliance enabling a broader spectrum of patients to be effectively treated.

The compounds of the present invention are surprisingly beneficial for two reasons: they have a dramatically lower PDE III inhibitory activity than 3-hydroxyanagrelide, yet still retain potent anti-megakaryocytic activity. Indeed these compounds have therapeutic indices which are likely to be much more favorable than that for anagrelide itself.

According to one aspect of the present invention, there is provided a compound of Formula (I) or a pharmaceutically acceptable salt or solvate thereof: 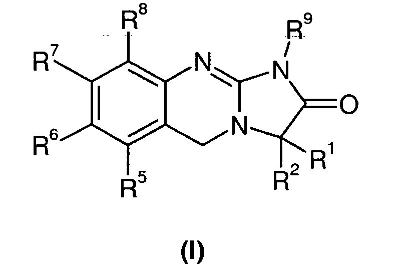 wherein:
R¹ and R² are each methyl or together form methylene; or R¹ and R², taken together with the carbon to which they are attached, form cyclopropyl;
two, three or four of R⁵, R⁶, R⁷ and R⁸ are hydrogen, and the remainder selected from R^{a} and R^{b};
R⁹ is hydrogen, C₁₋₆ alkyl or a Group I or Group II metal ion;
R^{a} is selected from C₁₋₆ alkyl and C₂₋₆ alkenyl, either of which is optionally substituted with 1, 2, 3, 4 or 5 R^{b};
R^{b} is selected from halo, trifluoromethyl, cyano, nitro, -OR^{c}, -C(O)R^{c}, -C(O)OR^{c}, -OC(O)R^{c}, -S(O)₁R^{c}, -N(R^{c})R^{d}, -C(O)N(R^{c})R ^{d}, -N(R^{c})C(O)R^{d}, -S(O)₁N(R^{c})R^{d} and -N(R^{c})S(O)₁R^{d};
R^{c} and R^{d} are each independently hydrogen or R^{e};
R^{e} is selected from C₁₋₆ alkyl and C₂₋₆ alkenyl, either of which is optionally substituted with 1, 2, 3, 4 or 5 substituents independently selected from halo, cyano, amino, hydroxy, nitro and C₁₋₆ alkoxy; and
1 is 0, 1 or 2.

In an embodiment, R¹ and R² are each methyl or together form methylene; or R¹ and R², taken together with the carbon to which they are attached, form cyclopropyl.

In another embodiment, R¹ and R² are each methyl or R¹ and R², taken together with the carbon to which they are attached, form cyclopropyl.

In an embodiment one or more of the following provisos apply:
(i) when the compound is of the following formula: 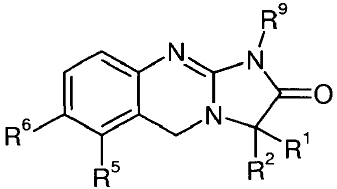 then R⁵ and R⁶ are not each halo; and
(ii) the compound is not one of the following compounds:

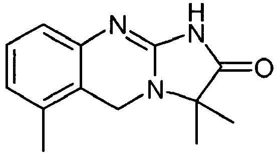

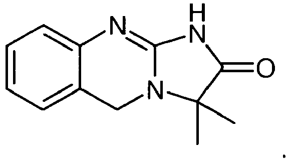

In an embodiment the compound is of the following Formula: 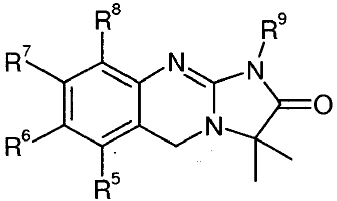 or a pharmaceutically acceptable salt or solvate thereof.

In an embodiment the compound is of the following Formula: 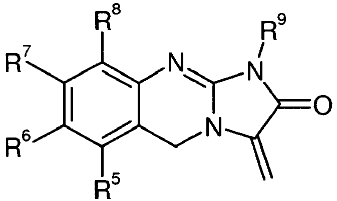 or a pharmaceutically acceptable salt or solvate thereof.

In an embodiment the compound is of the following Formula: 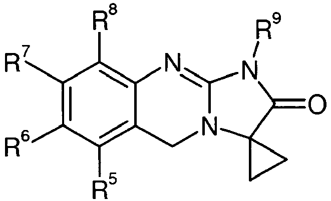 or a pharmaceutically acceptable salt or solvate thereof.

In an embodiment R⁹ is H. In another embodiment, R⁹ is hydrogen, C₁₋₆ alkyl or a Group I metal ion. In an alternative embodiment, R⁹ is C₁₋₆ alkyl and, in this case, the PDE III inhibiting activity is effectively eliminated. Me represents a particularly preferred alkyl substituent. In another alternative embodiment, R⁹ is a Group I metal ion and, in this case the compounds show significantly improved water solubility. Sodium represents a particularly preferred Group I metal.

According to Formula (I), two or three of R⁵, R⁶, R⁷ and R⁸ are hydrogen, and the remainder selected from R^{a} and R^{b}.

In an embodiment R^{a} is C₁₋₆ alkyl optionally substituted with 1, 2, 3, 4 or 5 R^{b}.

In an embodiment R^{a} is C₁, C₂, C₃ or C₄ alkyl, any of which is optionally substituted with 1, 2 or 3 R^{b}.

In an embodiment R^{b} is selected from halo, trifluoromethyl, cyano, nitro, -OR^{c}, -C(O)R^{c}, -C(O)OR^{c}, -OC(O)R^{c}, -S(O)₁R^{c}, -N(R^{c})R^{d}, -C(O)N(R^{c})R^{d}, -N(R^{c})C(O)R^{d}, -S(O)₁N(R^{c})R^{d} and -N(R^{c})S(O)₁R^{d}; wherein R^{c} and R^{d} are each independently hydrogen or C₁₋₆ alkyl optionally substituted with 1, 2, 3, 4 or 5 substituents independently selected from halo, cyano, amino, hydroxy, nitro and C₁₋₆ alkoxy.

In an embodiment R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy.

In an embodiment, three of R⁵, R⁶, R⁷ and R⁸ are hydrogen, and the other is selected from R^{a} and R^{b}. Of mention are compounds in which R⁷ and R⁸ are each hydrogen.

In an embodiment the compound is of one of the following Formulae: 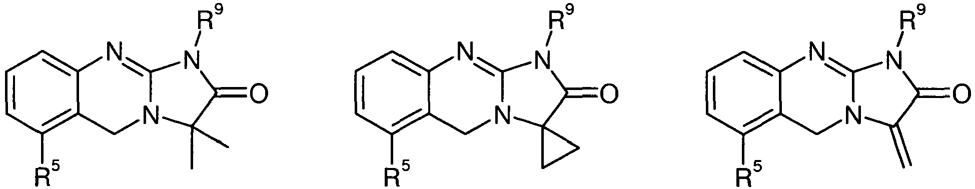 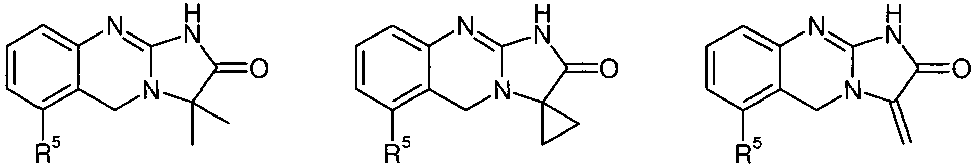 wherein R⁵ is selected from R^{a} and R^{b};
or, in each case, a pharmaceutically acceptable salt or solvate thereof.

With regard to each of the above Formulae, R⁵ may be, for example, selected from R^{a} and R^{b}; wherein R^{a} is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁵ is selected from fluoro, chloro, bromo, iodo, cyano, nitro, methyl, methoxy, trifluoromethyl, trifluoromethoxy, carboxylic acid, aminomethyl, fluoromethyl, chloromethyl, bromomethyl, dihalomethyl and methylsulphonyl.

In an embodiment the compound is of one of the following Formulae: 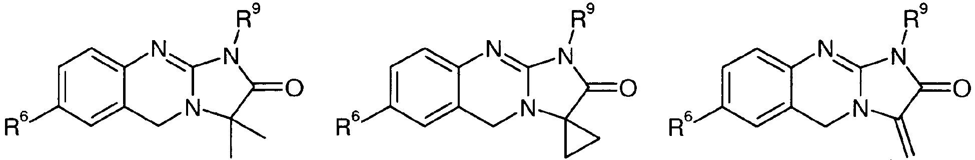 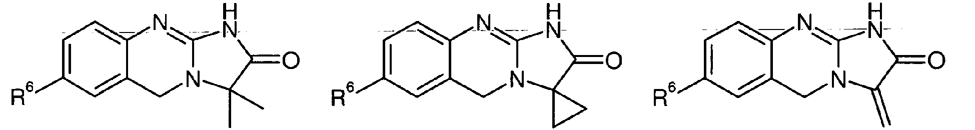 wherein R⁶ is selected from R^{a} and R^{b};
or, in each case, a pharmaceutically acceptable salt or solvate thereof.

With regard to each of the above Formulae, R⁶ may be, for example, selected from R^{a} and R^{b}; wherein R³ is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁶ is selected from fluoro, chloro, bromo, iodo, cyano, nitro, methyl, methoxy, trifluoromethyl, tiifluoromethoxy, carboxylic acid, aminomethyl, fluoromethyl, chloromethyl, bromomethyl, dihalomethyl and methylsulphonyl.

In an embodiment the compound is of one of the following Formulae: 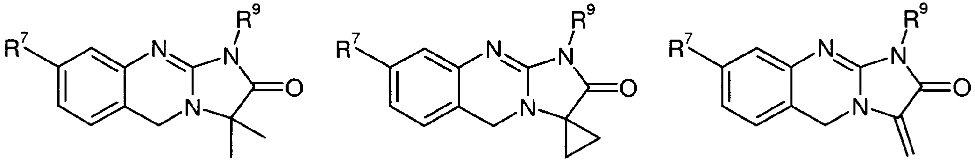 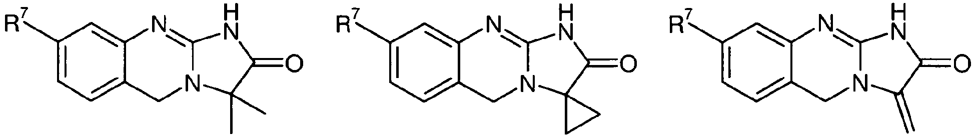 wherein R⁷ is selected from R^{a} and R^{b};
or, in each case, a pharmaceutically acceptable salt or solvate thereof.

With regard to each of the above Formulae, R⁷ may be, for example, selected from R^{a} and R^{b}; wherein R^{a} is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁷ is selected from fluoro, chloro, bromo, iodo, cyano, nitro, methyl, methoxy, trifluoromethyl, trifluoromethoxy, carboxylic acid, aminomethyl, fluoromethyl, chloromethyl, bromomethyl, dihalomethyl and methylsulphonyl.

In an embodiment the compound is of one of the following Formulae: 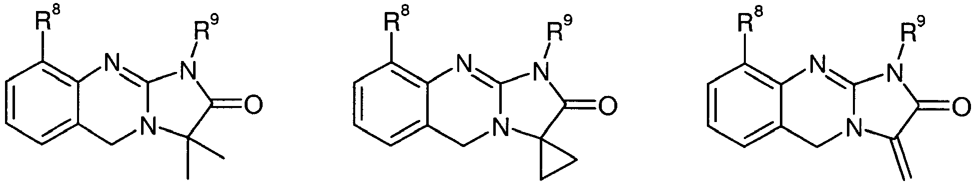 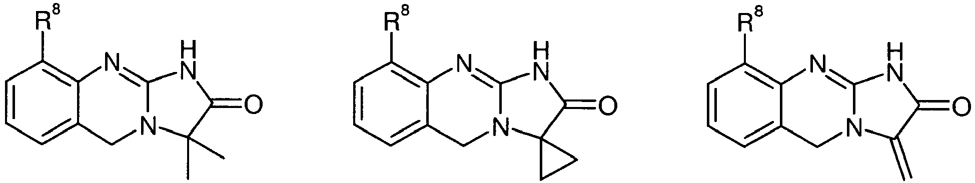 wherein R⁸ is selected from R^{a} and R^{b};
or, in each case, a pharmaceutically acceptable salt or solvate thereof.

With regard to each of the above Formulae, R⁸ may be, for example, selected from R^{a} and R^{b}; wherein R^{a} is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{e}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁸ is selected from fluoro, chloro, bromo, iodo, cyano, nitro, methyl, methoxy, trifluoromethyl, trifluoromethoxy, carboxylic acid, aminomethyl, fluoromethyl, chloromethyl, bromomethyl, dihalomethyl and methylsulphonyl.

In an embodiment, two of R⁵, R⁶, R⁷ and R⁸ are hydrogen, and the other two are independently selected from R^{a} and R^{b}. Of mention are compounds in which R⁷ and R⁸ are each hydrogen.

In an embodiment the compound is of one of the following Formulae: 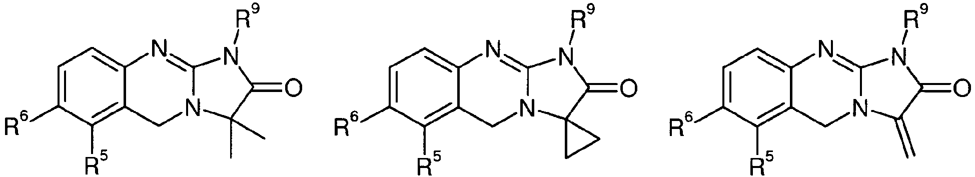 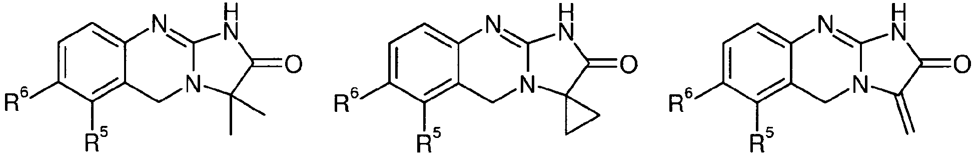 wherein R⁵ and R⁶ are each independently selected from R^{a} and R^{b};
or, in each case, a pharmaceutically acceptable salt or solvate thereof.

With regard to each of said Formulae, R⁵ and R⁶ may each be, for example, independently selected from R^{a} and R^{b}; wherein R^{a} is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁵ and R⁶ are each independently selected from fluoro, chloro, bromo, iodo, cyano, nitro, methyl, methoxy, trifluoromethyl, trifluoromethoxy, carboxylic acid, aminomethyl, fluoromethyl, chloromethyl, bromomethyl, dihalomethyl and methylsulphonyl.

In an embodiment, R⁵ is R^{f} and R⁶ is R^{g}, wherein R^{f} and R^{g} are as defined in the Table below:

| **R^{f}** | **R^{g}** |
|---|---|
| -CN | Halo |
| -OH | Halo |
| -N(O)₂ | Halo |
| C₁₋₆ alkyl | Halo |
| C₁₋₆ alkoxy | Halo |
| -C(O)OH | Halo |
| -S(O)₂-C₁₋₆ alkyl | Halo |
| Halo | -CN |
| -CN | -CN |
| -OH | -CN |
| -N(O)₂ | -CN |
| C₁₋₆ alkyl | -CN |
| C₁₋₆ alkoxy | -CN |
| -C(O)OH | -CN |
| -S(O)₂-C₁₋₆ alkyl | -CN |
| Halo | -OH |
| -CN | -OH |
| -OH | -OH |
| -N(O)₂ | -OH |
| C₁₋₆ alkyl | -OH |
| C₁₋₆ alkoxy | -OH |
| -C(O)OH | -OH |
| -S(O)₂-C₁₋₆ alkyl | -OH |
| Halo | -N(O)₂ |
| -CN | -N(O)₂ |
| -OH | -N(O)₂ |
| -N(O)₂ | -N(O)₂ |
| C₁₋₆ alkyl | -N(O)₂ |
| C₁₋₆ alkoxy | -N(O)₂ |
| -C(O)OH | -N(O)₂ |
| -S(O)₂-C₁₋₆ alkyl | -N(O)₂ |
| Halo | C₁₋₆ alkyl |
| -CN | C₁₋₆ alkyl |
| -OH | C₁₋₆ alkyl |
| -N(O)₂ | C₁₋₆ alkyl |
| C₁₋₆alkyl | C₁₋₆ alkyl |
| C₁₋₆ alkoxy | C₁₋₆ alkyl |
| -C(O)OH | C₁₋₆ alkyl |
| -S(O)₂-C₁₋₆ alkyl | C₁₋₆ alkyl |
| Halo | C₁₋₆alkoxy |
| -CN | C₁₋₆ alkoxy |
| -OH | C₁₋₆ alkoxy |
| -N(O)₂ | C₁₋₆ alkoxy |
| C₁₋₆ alkyl | C₁₋₆ alkoxy |
| C₁₋₆ alkoxy | C₁₋₆ alkoxy |
| -C(O)OH | C₁₋₆ alkoxy |
| -S(O)₂-C₁₋₆ alkyl | C₁₋₆ alkoxy |
| Halo | -C(O)OH |
| -CN | -C(O)OH |
| -OH | -C(O)OH |
| -N(O)₂ | -C(O)OH |
| C₁₋₆ alkyl | -C(O)OH |
| C₁₋₆ alkoxy | -C(O)OH |
| -C(O)OH | -C(O)OH |
| -S(O)₂-C₁₋₆ alkyl | -C(O)OH |
| Halo | -S(O)₂-C₁₋₆ alkyl |
| -CN | -S(O)₂-C₁₋₆ alkyl |
| -OH | -S(O)₂-C₁₋₆ alkyl |
| -N(O)₂ | -S(O)₂-C₁₋₆ alkyl |
| C₁₋₆ alkyl | -S(O)₂-C₁₋₆ alkyl |
| C₁₋₆ alkoxy | -S(O)₂-C₁₋₆ alkyl |
| -C(O)OH | -S(O)₂-C₁₋₆ alkyl |
| -S(O)₂-C₁₋₆ alkyl | -S(O)₂-C₁₋₆ alkyl |

In an embodiment the compound is of one of the following Formulae: 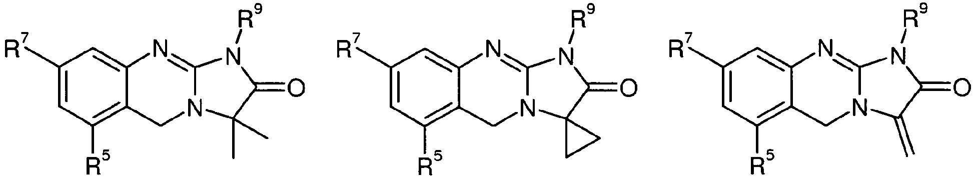 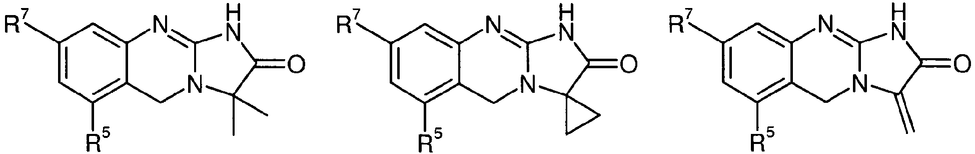 wherein R⁵ and R⁷ are each independently selected from R^{a} and R^{b};
or, in each case, a pharmaceutically acceptable salt or solvate thereof.

With regard to each of said Formulae, R⁵ and R⁷ may each be, for example, independently selected from R^{a} and R^{b}; wherein R^{a} is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁵ and R⁷ are each independently selected from fluoro, chloro, bromo, iodo, cyano, nitro, methyl, methoxy, trifluoromethyl, trifluoromethoxy, carboxylic acid, aminomethyl, fluoromethyl, chloromethyl, bromomethyl, dihalomethyl and methylsulphonyl.

In an embodiment, R⁵ is R^{f} and R⁷ is R^{g}, wherein R^{f} and R^{g} are as defined in the Table above or are each halo.

In an embodiment the compound is of one of the following Formulae: 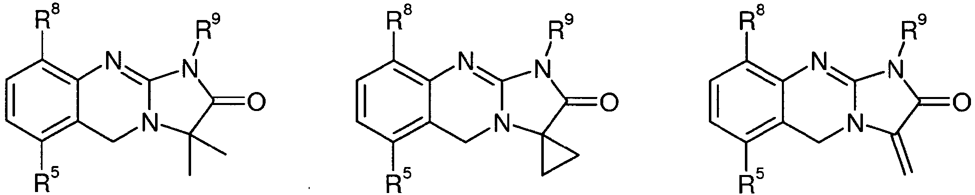 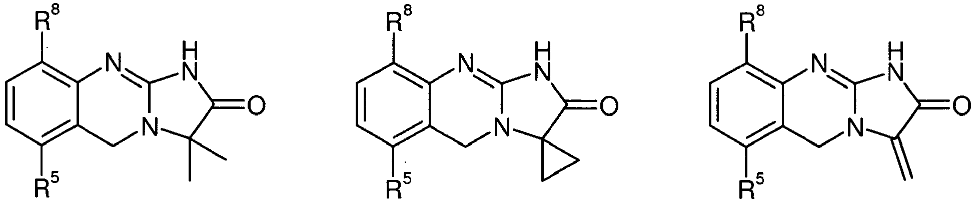 wherein R⁵ and R⁸ are each independently selected from R^{a} and R^{b};
or, in each case, a pharmaceutically acceptable salt or solvate thereof.

With regard to each of said Formulae, R⁵ and R⁸ may each be, for example, independently selected from R^{a} and R^{b}; wherein R^{a} is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁵ and R⁸ are each independently selected from fluoro, chloro, bromo, iodo, cyano, nitro, methyl, methoxy, trifluoromethyl, trifluoromethoxy, carboxylic acid, aminomethyl, fluoromethyl, chloromethyl, bromomethyl, dihalomethyl and methylsulphonyl.

In an embodiment, R⁵ is R^{f} and R⁸ is R⁸, wherein R^{f} and R^{g} are as defined in the Table above or are each halo.

In an embodiment the compound is of one of the following Formulae: 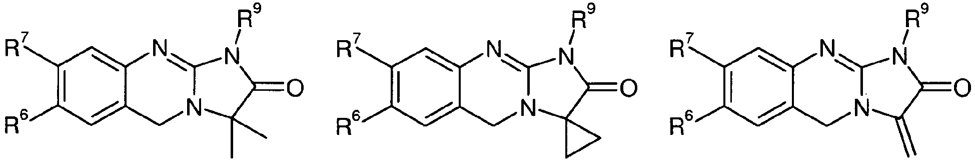 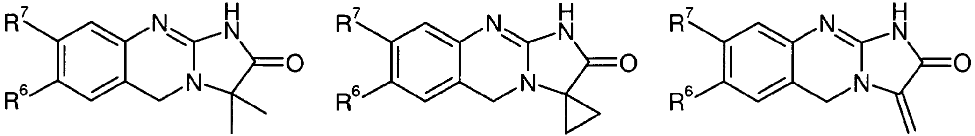 wherein R⁶ and R⁷ are each independently selected from R^{a} and R^{b};
or, in each case, a pharmaceutically acceptable salt or solvate thereof.

With regard to each of said Formulae, R⁶ and R⁷ may each be, for example, independently selected from R^{a} and R^{b}; wherein R^{a} is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁶ and R⁷ are each independently selected from fluoro, chloro, bromo, iodo, cyano, nitro, methyl, methoxy, trifluoromethyl, trifluoromethoxy, carboxylic acid, aminomethyl, fluoromethyl, chloromethyl, bromomethyl, dihalomethyl and methylsulphonyl.

In an embodiment, R⁶ is R^{f} and R⁷ is R^{g}, wherein R^{f} and R^{g} are as defined in the Table above or are each halo.

In an embodiment the compound is of one of the following Formulae: 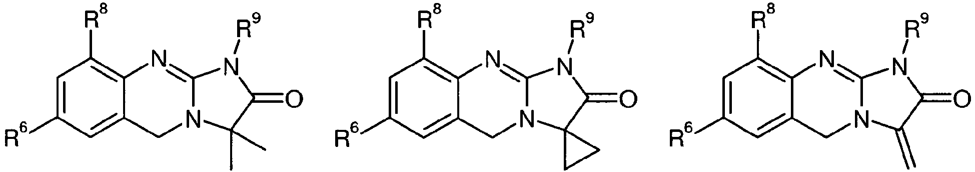 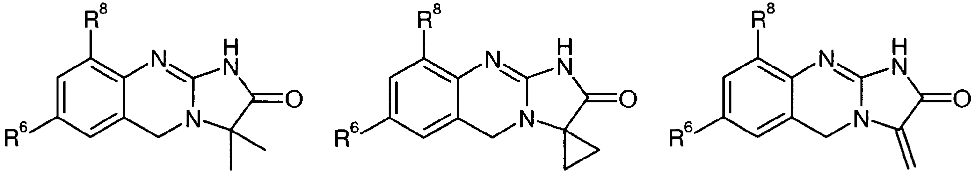 wherein R⁶ and R⁸ are each independently selected from R^{a} and R^{b};
or, in each case, a pharmaceutically acceptable salt or solvate thereof.

With regard to each of said Formulae, R⁶ and R⁸ may each be, for example, independently selected from R^{a} and R^{b}; wherein R^{a} is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁶ and R⁸ are each independently selected from fluoro, chloro, bromo, iodo, cyano, nitro, methyl, methoxy, trifluoromethyl, trifluoromethoxy, carboxylic acid, aminomethyl, fluoromethyl, chloromethyl, bromomethyl, dihalomethyl and methylsulphonyl.

In an embodiment, R⁶ is R^{f} and R⁸ is R^{g}, wherein R^{f} and R^{g} are as defined in the Table above or are each halo.

In an embodiment the compound is of one of the following Formulae: 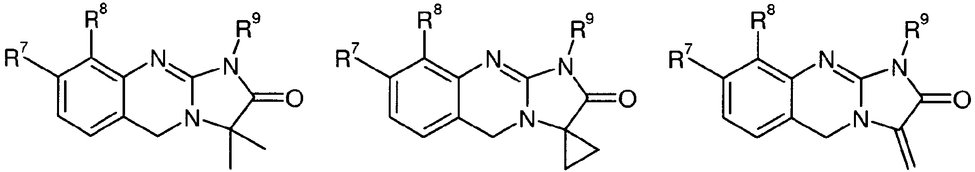 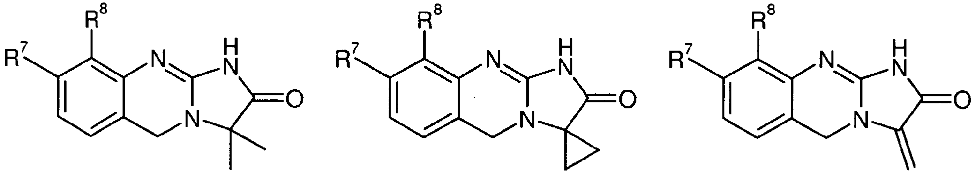 wherein R⁷ and R⁸ are each independently selected from R^{a} and R^{b};
or, in each case, a pharmaceutically acceptable salt or solvate thereof.

With regard to each of said Formulae, R⁷ and R⁸ may each be, for example, independently selected from R^{a} and R^{b}; wherein R^{a} is C₁₋₄ alkyl optionally substituted with 1, 2 or 3 R^{b}; and R^{b} is selected from fluoro, chloro, bromo, iodo, trifluoromethyl, cyano, nitro, -OR^{c}, -C(O)R^{c}, -C(O)OR^{c}, -OC(O)R^{c}, -S(O)₁R^{c} and -N(R^{c})R^{d}; wherein R^{c} and R^{d} are each independently hydrogen or C₁₋₄ alkyl optionally substituted with 1, 2 or 3 substituents independently selected from halo, cyano, amino, hydroxy, nitro and C₁₋₄ alkoxy. In an embodiment R⁷ and R⁸ are each independently selected from fluoro, chloro, bromo, iodo, cyano, nitro, methyl, methoxy, trifluoromethyl, trifluoromethoxy, carboxylic acid, aminomethyl, fluoromethyl, chloromethyl, bromomethyl, dihalomethyl and methylsulphonyl.

In an embodiment, R⁷ is R^{f} and R⁸ is R^{g}, wherein R^{f} and R^{g} are as defined in the Table above or are each halo.

In an embodiment, the compound is of the following Formula: 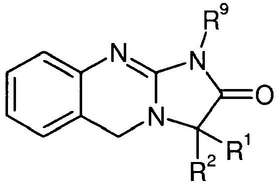 or a pharmaceutically acceptable salt or solvate thereof.

In an embodiment the compound is of the following Formula: 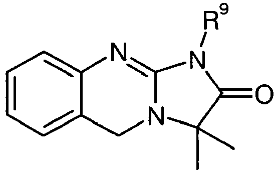 or a pharmaceutically acceptable salt or solvate thereof.

In an embodiment the compound is of the following Formula: 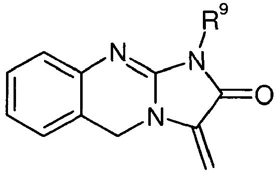 or a pharmaceutically acceptable salt or solvate thereof.

In an embodiment the compound is of the following Formula: 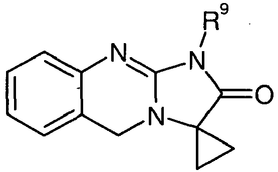 or a pharmaceutically acceptable salt or solvate thereof.

In a particular embodiment, the compound is selected from the following compounds:

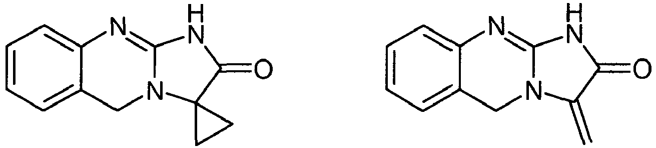

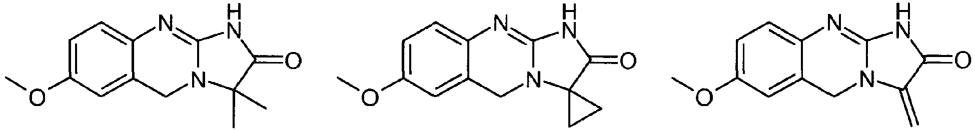

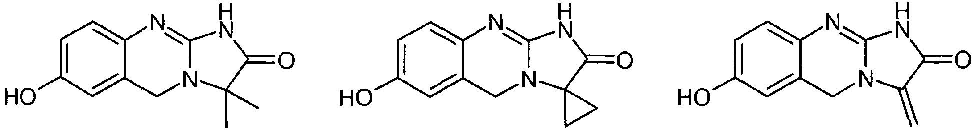

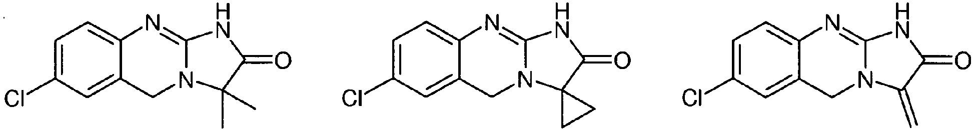

In an embodiment R⁹ is hydrogen, C₁₋₆ alkyl or a Group I metal ion.

It has also been found that the individual enantiomers of the present compounds show efficacy. The present invention therefore also relates to both the resolved optical isomers of such compounds as well as mixtures of enantiomers. For the purposes of comparison of the compounds of the present invention with anagrelide, the correct comparison is that made with the PDE III inhibitory activity of the 3-hydroxy metabolite of anagrelide since this is the predominant component in plasma after anagrelide treatment.

Regarding the use of the compounds of the invention in humans, there is provided:
a pharmaceutical composition comprising a compound of formula (I), or a pharmaceutically acceptable salt or solvate thereof, together with a pharmaceutically acceptable diluent or carrier, which may be adapted for oral, parenteral or topical administration;
a compound of formula (I), or a pharmaceutically acceptable salt or solvate thereof, or a pharmaceutical composition containing any of the foregoing, for use as a medicament;
the use of a compound of formula (I), or a pharmaceutically acceptable salt or solvate thereof in the manufacture of a medicament for the treatment of a disease selected from: myeloprolific diseases and/or generalised thrombotic diseases;

Separately, we have found that both (R) and (S) compounds show good anti-megakaryocytic activity whilst showing significantly reduced PDE III inhibition relative to 3-OH anagrelide. We thus expect that the compounds will have utility in treating myeloproliferative diseases.

Accordingly, the invention also includes the use of a compound of the invention, or a pharmaceutically acceptable salt or solvate thereof in the manufacture of a medicament for the treatment of myeloprolific diseases.

The present invention also encompasses pharmaceutical compositions comprising a compound or pharmaceutically acceptable salt of a compound of the present invention and a pharmaceutically acceptable carrier.

### DETAILED DESCRIPTION OF THE INVENTION

The present invention is directed to 3-substituted analogues of the established platelet lowering agent anagrelide. Substitution at the 3-position of the anagrelide molecule would be expected to block or hinder the principal site of metabolism and potentially preclude the formation of the highly potent PDE II inhibitor 3-OH anagrelide while substitution at the 1-position has surprisingly been found to abolish PDE III inhibition. The compounds of the present invention retain the anti-megakaryocytic properties (hence platelet lowering activity) of the parent drug molecule but have reduced PDE III inhibitory properties and hence lower potential for unwanted cardiovascular and anti-aggregatory side-effects. They also have the potential for improved pharmacokinetic characteristics as the result of inhibition of metabolism.

The pharmaceutically acceptable acid addition salts of certain of the compounds of formula (I) may also be prepared in a conventional manner. For example, a solution of the free base is treated with the appropriate acid, either neat or in a suitable solvent, and the resulting salt isolated either by filtration or by evaporation under reduced pressure of the reaction solvent. For a review on suitable salts, see "Handbook of Pharmaceutical Salts: Properties, Selection, and Use" by Stahl and Wermuth (Wiley-VCH, Weinheim, Germany, 2002).

### Definition of terms:-

Halo means a group selected from: fluoro, chloro, bromo or iodo.

The term "alkyl" as used herein as a group or a part of a group refers to a straight or branched hydrocarbon chain containing the specified number of carbon atoms. For example, C₁₋₁₀ alkyl means a straight or branched alkyl containing at least 1 and at most 10 carbon atoms. Examples of "alkyl" as used herein include, but are not limited to, methyl, ethyl, n-propyl, n-butyl, n-pentyl, isobutyl, isopropyl, t-butyl, hexyl, heptyl, octyl, nonyl and decyl. A C₁₋₄ alkyl group is one embodiment, for example methyl, ethyl, n-propyl, isopropyl, n-butyl, isobutyl or t-butyl. The term "alkoxy" as used herein refers to a straight or branched hydrocarbon chain group containing oxygen and the specified number of carbon atoms. For example, C₁₋₆ alkoxy means a straight or branched alkoxy containing at least 1 and at most 6 carbon atoms. Examples of "alkoxy" as used herein include, but are not limited to, methoxy, ethoxy, propoxy, prop-2-oxy, butoxy, but-2-oxy, 2-methylprop-1-oxy, 2-methylprop-2-oxy, pentoxy and hexyloxy. A C₁₋₄ alkoxy group is one embodiment, for example methoxy, ethoxy, propoxy, prop-2-oxy, butoxy, but-2-oxy or 2-methylprop-2-oxy.

The term "alkenyl" as used herein as a group or a part of a group refers to a straight or branched hydrocarbon chain containing the specified number of carbon atoms and containing at least one double bond. For example, the term "C₂₋₆ alkenyl" means a straight or branched alkenyl containing at least 2 and at most 6 carbon atoms and containing at least one double bond. Examples of "alkenyl" as used herein include, but are not limited to, ethenyl, 2-propenyl, 3-butenyl, 2-butenyl, 2-pentenyl, 3-pentenyl, 3-methyl-2-butenyl, 3-methylbut-2-enyl, 3-hexenyl and 1,1-dimethylbut-2-enyl. It will be appreciated that in groups of the form -O-C₂₋₆ alkenyl, the double bond is preferably not adjacent to the oxygen.

The compounds of the invention, i.e. those of formula (I), possess antimegakaryocytic activity in humans. They may be particularly useful in the treatment of myeloprolific diseases. The compounds may also find utility in the treatment of generalised thrombotic diseases.

It is to be appreciated that references to treatment include prophylaxis as well as the alleviation of established symptoms of a condition. "Treating" or "treatment" of a state, disorder or condition includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

Myeloproliferative diseases which may be treatable with the compounds of the present invention include: essential thrombocythemia, polycythema vera, chronic idiopathic myelofibrosis, chronic myeloid leukaemia with residual thrombocytosis, reactive thrombocytosis immediately preceding a surgical procedure, as an immediate or post operative preventative measures to minimise the risk of thrombus formation during or post surgery.

Thrombotic cardiovascular diseases (TCVD) (i.e. patients at increased generalised thrombotic risk) which may also be treatable with the compounds of the present invention include: myocardial infarct (heart attack), thrombotic stroke, patients having undergone coronary stent placement.

The compounds of the present invention may find utility for the reduction of atherothrombotic events as follows: recent MI, recent stroke or established peripheral arterial disease, acute coronary syndrome (unstable angina/non-Qwave MI), cardiovascular death, MI, stroke, and refractory ischemia.

It is to be understood that compounds of formula (I) may contain one or more asymmetric carbon atoms, thus compounds of the invention can exist as two or more stereoisomers.

Included within the scope of the present invention are all stereoisomers such as enantiomers and diastereomers, all geometric isomers and tautomeric forms of the compounds of formula (I), including compounds exhibiting more than one type of isomerism, and mixtures of one or more thereof.

Unexpectedly it has been found that stable metal salts can be prepared following deprotonation at the 1-position of the quinazoline ring structure. The value of such salts is seen in their relatively much greater aqueous solubility than the corresponding HBr salts. This is likely to facilitate the rapid dissolution and quantitative absorption of these generally poorly water soluble compounds and so represent a major clinical advantage. These salts are Group 1 metal salts and most usually are sodium or potassium salts.

Geometric isomers may be separated by conventional techniques well known to those skilled in the art, for example, by chromatography and fractional crystallisation.

Stereoisomers may be separated by conventional techniques known to those skilled in the art - see, for example, "Stereochemistry of Organic Compounds" by E L Eliel (Wiley, New York, 1994).

The compounds of formula I can be prepared using literature techniques and in an analogous manner to those described in Formula Scheme I and Formula Scheme II in US 4256748. By way of illustration, and without limitation, a compound of the invention may be obtained according to the following reaction scheme (in which R is, for example, ethyl or other alkyl):

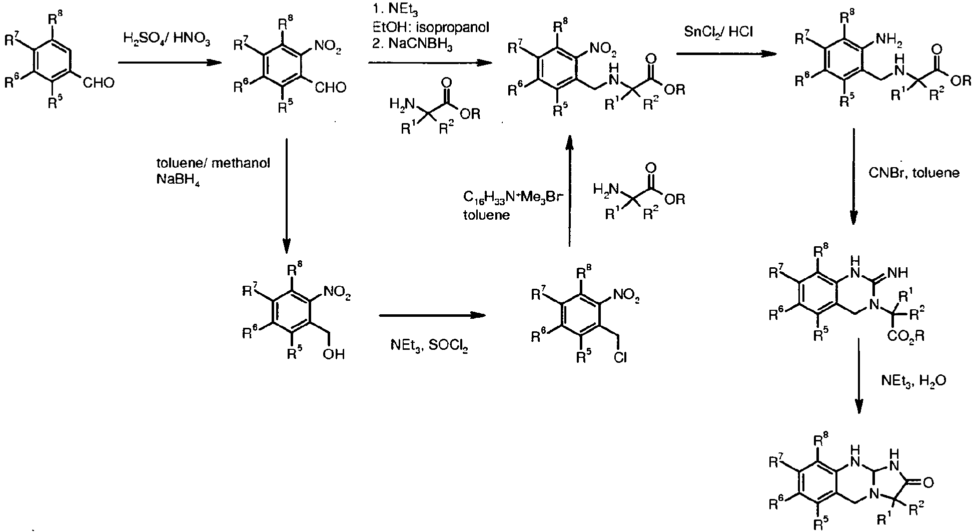

A person skilled in the art will be aware of variations of, and alternatives to, the process referred to above and to those in US 4256748 which allow the individual compounds defined by formula (I) to be obtained.

It will also be appreciated by a person skilled in the art that the compounds of the invention could be made by adaptation of the methods herein described and/or adaptation of methods known in the art, for example the art described herein, or using standard textbooks such as "Comprehensive Organic Transformations - A Guide to Functional Group Transformations", RC Larock, Wiley-VCH (1999 or later editions), "March's Advanced Organic Chemistry - Reactions, Mechanisms and Structure", MB Smith, J. March, Wiley, (5th edition or later) "Advanced Organic Chemistry, Part B, Reactions and Synthesis", FA Carey, RJ Sundberg, Kluwer Academic/Plenum Publications, (2001 or later editions), "Organic Synthesis - The Disconnection Approach", S Warren (Wiley), (1982 or later editions), "Designing Organic Syntheses" S Warren (Wiley) (1983 or later editions), "Guidebook To Organic Synthesis" RK Mackie and DM Smith (Longman) (1982 or later editions), etc.,and the references therein as a guide.

It will also be apparent to a person skilled in the art that sensitive functional groups may need to be protected and deprotected during synthesis of a compound of the invention. This may be achieved by conventional methods, for example as described in "Protective Groups in Organic Synthesis" by TW Greene and PGM Wuts, John Wiley & Sons Inc (1999), and references therein.

Compounds of the invention intended for pharmaceutical use may be administered as crystalline or amorphous products. They may be obtained, for example, as solid plugs, powders, or films by methods such as precipitation, crystallization, freeze drying, or spray drying, or evaporative drying. Microwave or radio frequency drying may be used for this purpose.

They may be administered alone or in combination with one or more other compounds of the invention or in combination with one or more other drugs. Generally, they will be administered as a formulation in association with one or more pharmaceutically acceptable excipients. Pharmaceutically acceptable excipients include one or more of: anti-oxidants, colourants, flavouring agents, preservatives and taste-masking agents.

Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995). The formulation of tablets is discussed in "Pharmaceutical Dosage Forms: Tablets, Vol. 1", by H. Lieberman and L. Lachman, Marcel Dekker, N.Y., N.Y., 1980 (ISBN 0-8247-6918-X).

The methods by which the compounds may be administered include oral administration by capsule, bolus, tablet, powders, lozenges, chews, multi and nanoparticulates, gels, solid solution, films, sprays, or liquid formulation. Liquid forms include suspensions, solutions, and syrups. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid preparation, for example, from a sachet.

The compounds may also be administered topically to the skin or mucosa, that is dermally or transdermally. Typical formulations for this purpose include pour-on solutions, sprays, powder formulations, gels, hydrogels, lotions, creams, ointments, films and patches, and implants.

The compounds can also be administered parenterally, or by injection directly into the blood stream, muscle or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Formulations may be immediate and/or modified controlled release. Controlled release formulations include Modified release formulations include: delayed-, sustained-, and pulsed-release.

### Dosages

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

In general however a suitable dose will be in the range of from about 0.001 to about 50 mg/kg of body weight per day, in a further embodiment, of from about 0.001 to about 5 mg/kg of body weight per day; in a further embodiment of from about 0.001 to about 0.5 mg/kg of body weight per day and in yet a further embodiment of from about 0.001 to about 0.1mg/kg of body weight per day. In further embodiments, the ranges can be of from about 0.1 to about 750 mg/kg of body weight per day, in the range of 0.5 to 60 mg/kg/day, and in the range of 1 to 20 mg/kg/day.

The desired dose may conveniently be presented in a single dose or as divided doses administered at appropriate intervals, for example as one, two, three, four or more doses per day. If the compounds are administered transdermally or in extended release form, the compounds could be dosed once a day or less.

The compound is conveniently administered in unit dosage form; for example containing 0.1 to 50 mg, conveniently 0.1 to 5 mg, most conveniently 0.1 to 5 mg of active ingredient per unit dosage form. In yet a further embodiment, the compound can conveniently administered in unit dosage form; for example containing 10 to 1500 mg, 20 to 1000 mg, or 50 to 700 mg of active ingredient per unit dosage form.

## Claims

1. A compound of Formula (I) or a pharmaceutically acceptable salt or solvate thereof: 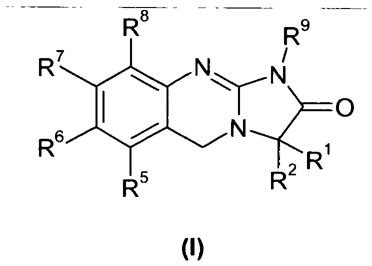 wherein:
R¹ and R² are each methyl or together form methylene; or R¹ and R², taken together with the carbon atom to which they are attached, form cyclopropyl;
two, three or four of R⁵, R⁶, R⁷ and R⁸ are hydrogen, and the remainder selected from R^{a} and R^{b};
R⁹ is hydrogen, C₁₋₆ alkyl or a Group I or Group II metal ion;
R^{a} is selected from C₁₋₆ alkyl and C₂₋₆ alkenyl, either of which is optionally substituted with 1, 2, 3, 4 or 5 R^{b};
R^{b} is selected from halo, trifluoromethyl, cyano, nitro, -OR^{c}, -C(O)R^{c}, -C(O)OR^{c}, -OC(O)R^{c}, -S(O)_{I}R^{c}, -N(R^{c})R^{d}, -C(O)N(R^{c})R^{d}, -N(R^{c})C(O)R^{d}, -S(O)_{I}N(R^{c})R^{d} and -N(R^{c})S(O)_{I}R^{d};
R^{c} and R^{d} are each independently hydrogen or R^{e};
R^{e} is selected from C₁₋₆ alkyl and C₂₋₆ alkenyl, either of which is optionally substituted with 1, 2, 3, 4 or 5 substituents independently selected from halo, cyano, amino, hydroxy, nitro and C₁₋₆ alkoxy; and
I is 0, 1 or 2;
and wherein each of the following provisos applies:
(i) when the compound is of the following formula: 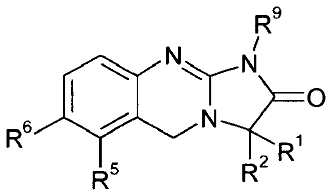 then R⁵ and R⁶ are not each halo; and
(ii) the compound is not one of the following compounds:

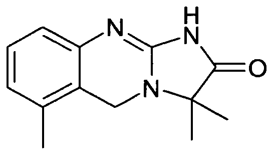

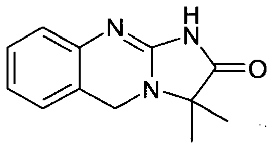

2. A compound according to claim 1, wherein R¹ and R² are each methyl.

3. A compound according to claim 1, wherein R¹ and R² taken together with the carbon atom to which they are attached, form cyclopropyl.

4. A compound according to any preceding claim, wherein two of R⁵, R⁶, R⁷ and R⁸ are hydrogen, and the other two are independently selected from R^{a} and R^{b}.

5. A compound according to claim 4, wherein R⁷ and R⁸ are each hydrogen.

6. A compound according to any of claims 1 to 3, wherein three of R⁵, R⁶, R⁷ and R⁸ are hydrogen, and the other is selected from R^{a} and R^{b};

7. A compound according to claim 6, wherein R⁷ and R⁸ are each hydrogen.

8. A compound according to any of claims 1 to 3, wherein R⁵, R⁶, R⁷ and R⁸ are each hydrogen.

9. A compound according to any preceding claim, wherein R⁹ is hydrogen, methyl or sodium.

10. A compound according to claim 9, wherein R⁹ is hydrogen.

11. A compound according to any preceding claim, wherein R^{a} is C₁₋₆ alkyl optionally substituted with 1, 2, 3, 4 or 5 R^{b}; and R^{b} is selected from halo, cyano, nitro, -OH, C₁₋₆ alkoxy, -C(O)OH and -S(O)₂-C₁₋₆ alkyl.

12. A pharmaceutical composition comprising a compound of any of claims 1 to 11, or a pharmaceutically acceptable salt or solvate thereof, together with a pharmaceutically acceptable diluent or carrier, which may be adapted for oral, parenteral or topical administration.

13. A compound of formula (I) as defined in any of claims 1 to 11, or a pharmaceutically acceptable salt or solvate thereof, or a pharmaceutical composition containing any of the foregoing, for use as a medicament.

14. A compound of formula (I) as defined in any of claims 1 to 11, or a pharmaceutically acceptable salt or solvate thereof, or a pharmaceutical composition containing any of the foregoing, for use in the treatment of a disease selected from: myeloprolific diseases and generalised thrombotic diseases.

15. Use of a compound of formula (I) as defined in any of claims 1 to 11 for the reduction of platelet count.

## Patentansprüche

1. Verbindung der Formel (I) oder ein pharmazeutisch unbedenkliches Salz oder Solvat davon:
wobei:
R¹ und R² jeweils Methyl sind oder zusammen Methylen bilden; oder R¹ und R² zusammen genommen mit dem Kohlenstoffatom, an das sie angelagert sind, Cyclopropyl bilden;
zwei, drei oder vier von R⁵, R⁶, R⁷ und R⁸ Wasserstoff sind und der Rest aus R^{a} und R^{b} ausgewählt ist;
R⁹ Wasserstoff, C₁₋₆-Alkyl oder ein Ion eines Metalls der Gruppe I oder der Gruppe II ist;
R^{a} aus C₁₋₆-Alkyl und C₂₋₆-Alkenyl ausgewählt ist, die beide gegebenenfalls mit 1, 2, 3, 4 oder 5 R^{b} substituiert sind;
R^{b} aus Halo, Trifluormethyl, Cyano, Nitro, -OR^{c}, -C(O)R^{c}, -C(O)OR^{c}, -OC(O)R^{c}, -S(O)₁R^{c}, - N(R^{c})R^{d}, -C(O)N(R^{c})R^{d}, -N(R^{c})C(O)R^{d}, -S(O)₁N(R^{c})R^{d} und -N(R^{c})S(O)₁R^{d} ausgewählt ist;
R^{c} und R^{d} jeweils unabhängig Wasserstoff oder R^{e} sind;
R^{e} aus C₁₋₆-Alkyl und C₂₋₆-Alkenyl ausgewählt ist, die beide gegebenenfalls mit 1, 2, 3, 4 oder 5 Substituenten substituiert sind, die unabhängig aus Halo, Cyano, Amino, Hydroxy, Nitro und C₁₋₆-Alkoxy ausgewählt sind;
10, 1 oder 2 ist;
und wobei jede der folgenden Maßgaben gilt:
(i) wenn die Verbindung die folgende Formel aufweist: 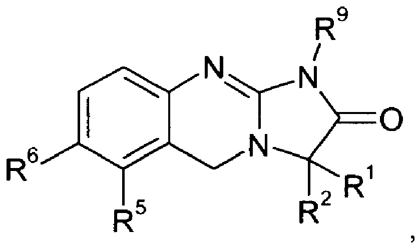 sind R⁵ und R⁶ jeweils nicht Halo; und
(ii) die Verbindung ist keine der folgenden Verbindungen:

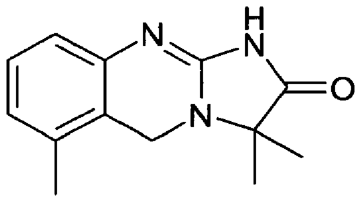

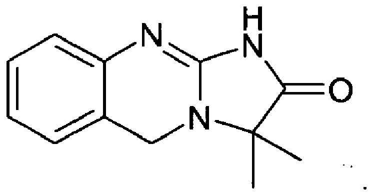

2. Verbindung nach Anspruch 1, wobei R¹ und R² jeweils Methyl sind.

3. Verbindung nach Anspruch 1, wobei R¹ und R² zusammen genommen mit dem Kohlenstoffatom, an das sie angelagert sind, Cyclopropyl bilden.

4. Verbindung nach einem der vorhergehenden Ansprüche, wobei zwei von R⁵, R⁶, R⁷ und R⁸ Wasserstoff sind und die anderen zwei unabhängig aus R^{a} und R^{b} ausgewählt sind.

5. Verbindung nach Anspruch 4, wobei R⁷ und R⁸ jeweils Wasserstoff sind.

6. Verbindung nach einem der Ansprüche 1 bis 3, wobei drei von R⁵, R⁶, R⁷ und R⁸ Wasserstoff sind und der andere aus R^{a} und R^{b} ausgewählt ist.

7. Verbindung nach Anspruch 6, wobei R⁷ und R⁸ jeweils Wasserstoff sind.

8. Verbindung nach einem der Ansprüche 1 bis 3, wobei R⁵, R⁶, R⁷ und R⁸ jeweils Wasserstoff sind.

9. Verbindung nach einem der vorhergehenden Ansprüche, wobei R⁹ Wasserstoff, Methyl oder Natrium ist.

10. Verbindung nach Anspruch 9, wobei R⁹ Wasserstoff ist.

11. Verbindung nach einem der vorhergehenden Ansprüche, wobei R^{a} C₁₋₆-Alkyl ist, das gegebenenfalls mit 1, 2, 3, 4 oder 5 R^{b} substituiert ist; und R^{b} aus Halo, Cyano, Nitro, -OH, C₁₋₆-Alkoxy, -C(O)OH und -S(O)₂-C₁₋₆-Alkyl ausgewählt ist.

12. Pharmazeutische Zusammensetzung, die eine Verbindung nach einem der Ansprüche 1 bis 11 oder ein pharmazeutisch unbedenkliches Salz oder Solvat davon zusammen mit einem pharmazeutisch unbedenklichen Verdünnungsmittel oder Trägerstoff umfasst, das bzw. der auf die orale, parenterale oder topische Verabreichung ausgerichtet sein kann.

13. Verbindung der Formel (I) nach einem der Ansprüche 1 bis 11 oder ein pharmazeutisch unbedenkliches Salz oder Solvat davon oder eine pharmazeutische Zusammensetzung, die eine beliebige bzw. ein beliebiges der vorhergehenden enthält, zur Verwendung als ein Arzneimittel.

14. Verbindung der Formel (I) nach einem der Ansprüche 1 bis 11 oder ein pharmazeutisch unbedenkliches Salz oder Solvat davon oder eine pharmazeutische Zusammensetzung, die eine beliebige bzw. ein beliebiges der vorhergehenden enthält, zur Verwendung bei der Behandlung einer Erkrankung, die aus den folgenden ausgewählt ist: myeloproliferative Erkrankungen und generalisierte thrombotische Erkrankungen.

15. Verwendung einer Verbindung der Formel (I) nach einem der Ansprüche 1 bis 11 zur Senkung der Thrombozytenzahl.

## Revendications

1. Composé de Formule (I) ou sel ou solvate pharmaceutiquement acceptable de celui-ci : 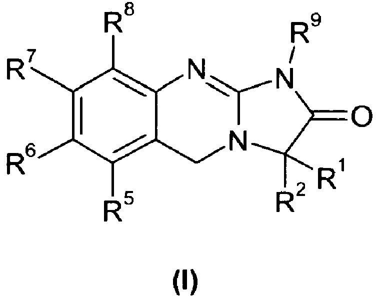 dans laquelle :
R¹ et R² sont chacun méthyle ou forment ensemble du méthylène ; ou R¹ et R², pris ensemble avec l'atome de carbone auquel ils sont reliés, forment du cyclopropyle ;
deux, trois ou quatre de R⁵, R⁶, R⁷ et R⁸ sont hydrogène et le reste sélectionné parmi R^{a} et
R^{b} ;
R⁹ est hydrogène, alkyle en C₁₋₆ ou un ion métallique du Groupe I ou Groupe II ;
R^{a} est sélectionné parmi alkyle en C₁₋₆ et alkényle en C₂₋₆ dont l'un ou l'autre est en option substitué par 1, 2, 3, 4 ou 5 R^{b} ;
R^{b} est sélectionné parmi halogéno, trifluorométhyle, cyano, nitro, -OR^{c}, -C(O)R^{c}, -C(O)OR^{c}, -OC(O)R^{c}, -S(O)₁R^{c}, -N(R^{c})R^{d}, -C(O)N(R^{c})R^{d}, -N(R^{c})C(O)R^{d}, -S(O)₁N(R^{c})R^{d} et - N(R^{c})S(O)₁R^{d} ;
R^{c} et R^{d} sont chacun indépendamment hydrogène ou R^{e} ;
R^{e} est sélectionné parmi alkyle en C₁₋₆ et alkényle en C₂₋₆ dont l'un ou l'autre est en option substitué par 1, 2, 3, 4 ou 5 substituants indépendamment sélectionnés parmi halogéno, cyano, amino, hydroxy, nitro et alcoxy en C₁₋₆ ;et 1 est 0, 1 ou 2 ;
et dans laquelle chacune des conditions suivantes s'applique :
(i) lorsque le composé est de la formule suivante : 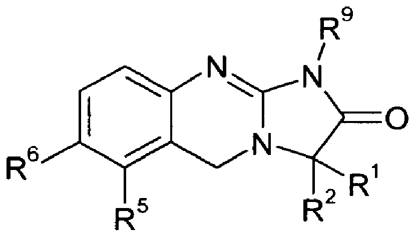 alors R⁵ et R⁶ ne sont pas chacun halogéno ; et
(ii) le composé n'est pas un des composés suivants :

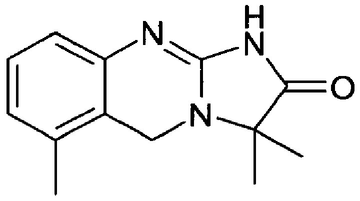

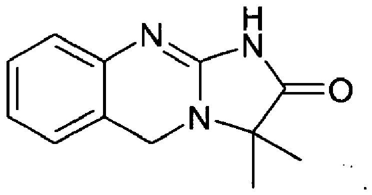

2. Composé selon la revendication 1, dans lequel R¹ et R² sont chacun méthyle.

3. Composé selon la revendication 1, dans lequel R¹ et R², pris ensemble avec l'atome de carbone auquel ils sont reliés, forment du cyclopropyle.

4. Composé selon l'une quelconque des revendications précédentes, dans lequel deux de R⁵, R⁶, R⁷ et R⁸ sont hydrogène, et les deux autres sont indépendamment sélectionnés parmi R^{a} et R^{b}.

5. Composé selon la revendication 4, dans lequel R⁷ et R⁸ sont chacun hydrogène.

6. Composé selon l'une quelconque des revendications 1 à 3, dans lequel trois de R⁵, R⁶, R⁷ et R⁸ sont hydrogène, et l'autre est sélectionné parmi R^{a} et R^{b}.

7. Composé selon la revendication 6, dans lequel R⁷ et R⁸ sont chacun hydrogène.

8. Composé selon l'une quelconque des revendications 1 à 3, dans lequel R⁵, R⁶, R⁷ et R⁸ sont chacun hydrogène.

9. Composé selon l'une quelconque des revendications précédentes, dans lequel R⁹ est hydrogène, méthyle ou sodium.

10. Composé selon la revendication 9, dans lequel R⁹ est hydrogène.

11. Composé selon l'une quelconque des revendications précédentes, dans lequel R^{a} est alkyle en C₁₋₆ substitué en option par 1, 2, 3, 4 ou 5 R^{b} ; et R^{b} est sélectionné parmi halogéno, cyano, nitro, -OH, alcoxy en C₁₋₆, -C(O)OH et -S(O)₂-C₁₋₆-alkyle.

12. Composition pharmaceutique comprenant un composé selon l'une quelconque des revendications 1 à 11 ou un sel ou solvate pharmaceutiquement acceptable de celui-ci, avec un diluant ou support pharmaceutiquement acceptable, qui peut être adaptée à l'administration par voie orale, parentérale ou topique.

13. Composé de formule (I) selon l'une quelconque des revendications 1 à 11 ou sel ou solvate pharmaceutiquement acceptable de celui-ci, ou composition pharmaceutique contenant l'un quelconque des composés précédents, destiné(e) à être utilisé(e) en tant que médicament.

14. Composé de formule (I) selon l'une quelconque des revendications 1 à 11 ou sel ou solvate pharmaceutiquement acceptable de celui-ci, ou composition pharmaceutique contenant l'un quelconque des composés précédents, destiné(e) à être utilisé(e) dans le traitement d'une maladie sélectionnée parmi : des maladies myéloprolifératives et des maladies thrombotiques généralisées.

15. Utilisation d'un composé de formule (I) selon l'une quelconque des revendications 1 à 11 pour la réduction de la numération des plaquettes.
